# EUROPEAN PATENT APPLICATION

(11) **EP 4 613 735 A1**
(43) Date of publication of application: **10.09.2025**
(21) Application number: 23884748.7
(22) Date of filing: 27.10.2023
(51) Int. Cl.: C07C 209/16, C07C 211/12, C07C 213/02, C07D 295/023

(54) **METHOD FOR PREPARING HEXAMETHYLENEDIAMINE**

(30) Priority: 31.10.2022 CN 202211350196; 31.10.2022 CN 202211351228; 20.10.2023 CN 202311368569
(71) Applicant: CHINA PETROLEUM & CHEMICAL CORPORATION, Chaoyang District Beijing 100728 (CN); Sinopec (Beijing) Research Institute of Chemical Industry Co., Ltd., Beijing 100013 (CN)
(72) Inventor: SHU, Zhan, Beijing 100013 (CN); GUO, Liang, Beijing 100013 (CN); LIU, Zhixin, Beijing 100013 (CN); LUO, Shujuan, Beijing 100013 (CN); LI, Dongfeng, Beijing 100013 (CN); WANG, Guoqing, Beijing 100013 (CN); FAN, Xiaozhe, Beijing 100013 (CN); TIAN, Jun, Beijing 100013 (CN); SHI, Qian, Beijing 100013 (CN); LI, Yi, Beijing 100013 (CN); LI, Yulong, Beijing 100013 (CN); ZHAO, Meng, Beijing 100013 (CN); SHAO, Huawei, Beijing 100013 (CN); SUN, Ruliu, Beijing 100013 (CN); ZHANG, Jingsheng, Beijing 100013 (CN); QU, Yuping, Beijing 100013 (CN); WANG, Chao, Beijing 100013 (CN); LI, YAN, Beijing 100013 (CN)
(74) Representative: Hoffmann Eitle
(86) International application number: PCT/CN2023/127038
(87) International publication number: WO 2024/093816

(57) **Abstract**

This invention discloses a method for preparing hexamethylenediamine, which comprises the following steps: (1) under an ammoniation reaction condition, optionally in the presence of a first solvent, subjecting hexanediol and ammonia to an ammoniation reaction to obtain an ammoniation reaction product; (2) separating the ammoniation reaction product from step (1) to obtain a hexamethylenediamine product and heavy components; and (3) under an ammonolysis reaction condition, subjecting the heavy components to ammonolysis to obtain an ammonolysis reaction product, and returning the ammonolysis reaction product to step (2). The invention not only may improve the yield of the hexamethylenediamine, but also may prolong the service life of a catalyst.

## Description

### Technical field

The invention relates to a technical field of hexamethylenediamine production, in particular to a method for preparing hexamethylenediamine from hexanediol as raw material.

### Background

Hexamethylenediamine is an important chemical raw material. It is an essential raw material monomer for the manufacture of polyamide nylon 66. It is also used to manufacture other types of polyamides such as nylon 610, nylon 612, etc. It is an important intermediate raw material in the engineering plastics industry. As the market scale of nylon 66 continues to expand, the demand for its raw material hexamethylenediamine is also growing continuously.

The main methods for industrial production of hexamethylenediamine include an adiponitrile method, a caprolactam method, an adipic acid method, a hexanediol ammoniation method, and the like.

The adiponitrile method is the most commonly used production method in industrial production. The method is divided into two approaches: a high-pressure method and a low-pressure method. The high-pressure method uses iron based and cobalt-copper based catalysts as the main catalysts, and the conversion rate may reach 95% or more, but the pressure is high and there is an extremely high requirement on equipments. The low-pressure method uses Raney nickel as the main catalyst, but there are problems related to the safety and stability of the Raney nickel catalyst, the supply of the adiponitrile raw material, etc.

In the caprolactam method, caprolactam is ammoniated with ammonia under the catalysis of a phosphate salt to produce 6-aminocapronitrile, which is hydrogenated to produce hexamethylenediamine. The key step of the method is the ammoniation of caprolactam. This method has been gradually abandoned because it is only applicable to small-scale production and its production cost is high.

The adipic acid method is that adipic acid is ammoniated with ammonia, followed by dehydration to produce adiponitrile, and adiponitrile is hydrogenated to obtain hexamethylenediamine. This method has high production cost and a long process, thus is limited in process technology development.

The hexanediol method is the preparation of hexamethylenediamine by subjecting hexanediol and ammonia, as raw materials, to ammoniation reaction. Hexanediol is a colorless, transparent liquid with low toxicity and little harm to the environment and human body. The preparation of hexamethylenediamine using hexanediol as raw material is a green and environmentally friendly route. However, in the prior art, the common problems in the process of ammoniation of hexanediol to produce hexamethylenediamine are low hexanediol conversion rate, hexamethylenediamine selectivity and hexamethylenediamine yield. As calculated according to the disclosed data, the yield of hexamethylenediamine is usually less than 50%, and the economic efficiency is left to be improved.

CN114433122A discloses a catalyst for preparing 1,6-hexamethylenediamine by ammoniation of 1,6-hexanediol in the presence of hydrogen. In a fixed bed reactor, the molar ratio of hydrogen: ammonia: 1,6-hexanediol is 3:8:1, the volume space velocity of 1,6-hexanediol is 0.6h⁻¹, the reaction temperature is 205°C, the reaction pressure is 13MPa, the conversion rate of hexanediol is about between 80% and 92%, and the selectivity of hexamethylenediamine is about 42%-52%. According to the disclosed data, it is calculated that the yield of hexamethylenediamine is about 34%-48%.

JP2022112396A discloses a method for producing hexamethylenediamine from 1,6-hexanediol, comprising producing hexamethylenediamine by amination reaction of 1,6-hexanediol under mild conditions in the presence of a solid catalyst, wherein the catalyst comprises an active component containing a metal element selected from elements of Group 8, Group 9, Group 10 and Group 11 in the periodic table and a carrier containing one or more metal elements selected from rare earth elements, and elements of Group 4 and Group 5. The examples show that the yield of hexamethylenediamine is less than 40%.

In addition, the process of preparing hexamethylenediamine by ammoniation of hexanediol in the prior art is easily involved in polymerization to produce organic amines with high carbon atoms, mainly dihexamethylenetriamine and other organic amines with carbon atoms greater than 12, which are collectively referred to as C12 and C12+ amines in the invention. If this part of product is not recycled, it may only be discharged as waste liquid, resulting in a low overall yield of the target product hexamethylenediamine. Therefore, there is still a need for a method for preparing hexamethylenediamine, in which hexamethylenediamine may be produced from hexanediol in a high yield, thereby improving the process economy.

### Summary

The object of the invention is to provide a method for preparing hexamethylenediamine with high yield so as to overcome the above problems existing in the prior art.

In order to achieve the above object, the invention provides a method for preparing hexamethylenediamine, which comprises the following steps:
(1) under an ammoniation reaction condition, optionally in the presence of a first solvent, subjecting hexanediol and ammonia to an ammoniation reaction to obtain an ammoniation reaction product;
(2) separating the ammoniation reaction product from step (1) to obtain a hexamethylenediamine product and heavy components; and
(3) under an ammonolysis reaction condition, subjecting the heavy components to ammonolysis to obtain an ammonolysis reaction product, and returning the ammonolysis reaction product to step (2).

It should be emphasized that the ammonolysis described in the invention refers to the cracking of high carbon amines into low carbon amines, specifically refers to the chemical reaction of the heavy components, i.e., C12 and C12+ amines defined above in the invention, under the action of a catalyst to produce low carbon amines such as hexamethylenediamine.

The method of the invention has the following beneficial effects:
(1) By ammonolysis of the heavy components from the ammoniation reaction, the invention not only improves the yield of hexamethylenediamine but also reduces the discharge amount of the waste liquid from the ammoniation reaction, thereby greatly reducing the production cost of hexamethylenediamine.
(2) The technological process of the invention is intrinsically safe, does not involve highly toxic nitrile chemicals, and the technical route is safe and environmentally friendly. The hexamethylenediamine product obtained by the invention has stable output, high purity, high yield and few impurities.
(3) For enterprises having hexanediol or upstream raw material resources, the invention provides a method for preparing hexamethylenediamine by ammoniation of an alcohol, which is a simple process flow, saves equipment investment, and has high economic added value of the product.

### Detailed description

The endpoint values of the ranges and any values disclosed herein are not limited to the precise ranges or values, and these ranges or values should be understood to include values approximating these ranges or values. For numerical ranges, one or more new numerical ranges may be obtained by combining the endpoint values of each range with each other, by combining the endpoint values of each range with individual point values, and by combining individual point values with each other, and these numerical ranges should be deemed to be specifically disclosed herein.

The invention provides a method for preparing hexamethylenediamine, which comprises the following steps:
(1) under an ammoniation reaction condition, optionally in the presence of a first solvent, subjecting hexanediol and ammonia to an ammoniation reaction to obtain an ammoniation reaction product;
(2) separating the ammoniation reaction product from step (1) to obtain a hexamethylenediamine product and heavy components; and
(3) under an ammonolysis reaction condition, subjecting the heavy components to ammonolysis to obtain an ammonolysis reaction product, and returning the ammonolysis reaction product to step (2).

According to the invention, a solvent (i.e., a first solvent) is not essential but optionally used in the ammoniation reaction of hexanediol and ammonia, thus the ammoniation reaction may be carried out in a solvent or in the absence of a solvent. In the prior art, ammoniation reaction is usually carried out at normal pressure or low pressure, and the ammonia is in a gas phase state, so a solvent needs to be added to dissolve the reaction stream ammonia gas in order to effectively carry out the ammoniation reaction. In the invention, the pressure of the ammoniation reaction is relatively high, and the ammonia is liquefied to be in a liquid phase state, thus the ammoniation reaction of hexanediol and liquid ammonia may be carried out in the absence of a solvent. The solvent added in the invention is to dissolve the ammoniation reaction raw materials and reaction products such as hexanediol, hexamethylenediamine, aminohexanol, hexamethyleneimine and N-(6-aminohexyl)hexamethyleneimine.

According to the invention, in the ammoniation reaction of hexanediol and ammonia, the molar ratio of ammonia to hexanediol is 10-90:1, preferably 10-80:1, such as 15-70:1, 15-60:1, and 20-45:1. The ammoniation reaction is carried out in the presence of hydrogen, and the molar ratio of hydrogen to hexanediol is 0.05-25:1, preferably 0.05-20:1, such as 0.1-10:1, 0.2-5:1, and 0.3-3:1. The molar ratio of ammonia, hydrogen and hexanediol refers to the molar ratio in the mixture at the inlet of the ammoniation reactor.

According to the invention, in the ammoniation reaction of hexanediol and ammonia, the temperature of the ammoniation reaction is 100-300°C, preferably 100-250°C, such as 120-250°C, 130-210°C, and 140-200°C. The pressure of the ammoniation reaction is 4-25 MPaG, preferably 6-20 MPaG, such as 6-18 MPaG, 8-16 MPaG, and 10-14 MPaG. The liquid volume space velocity of the fresh hexanediol is 0.01-15h⁻¹, preferably 0.01-10h⁻¹, such as 0.03-8h⁻¹, 0.05-4h⁻¹, and 0.1-2h⁻¹.

The ammoniation reaction catalyst used during the ammoniation reaction of the invention may be any type of catalyst in the art that can ammoniate hexanediol to produce hexamethylenediamine, for example, the ammoniation reaction catalysts in CN114433086A, CN114433087A, CN114433113A, etc. Preferably, the catalyst comprises a carrier, an active component supported on the carrier, and optionally an auxiliary agent, wherein the carrier comprises a doping element, alumina and an optional other carrier, wherein the other carrier is selected from at least one of silicon oxide, molecular sieves and diatomaceous earth. The pore volume of the carrier with a pore diameter less than 7.5 nm accounts for less than 20% of the pore volume of the carrier, the pore volume of the carrier with a pore diameter less than 9 nm accounts for less than 40% of the pore volume of the carrier, and the pore volume of the carrier with a pore diameter greater than 27 nm accounts for less than 5% of the pore volume of the carrier. The ammonia adsorption capacity of the carrier is 0.3-0.6 mmol/g. The L acid of the carrier accounts for more than 90% of the sum of the L acid and the B acid. The active component is cobalt and/or nickel.

According to the ammoniation reaction catalyst of the invention, preferably, the carrier is selected from alumina incorporated with at least one of silicon oxide, molecular sieves and diatomaceous earth, and alumina without incorporation. **The** content of the alumina carrier in the carrier accounts for 65 wt% or more, preferably 75 wt% or more of the total amount of the alumina carrier and other carriers.

According to the ammoniation reaction catalyst of the invention, preferably, the carrier may further comprise a doping element, and the content of the doping element accounts for 0.05-3wt%, more preferably 0.08-2wt%, and further preferably 0.1-1.5wt%, of the total weight of the components other than the doping element in the carrier. **The** components other than the doping element mainly refer to the alumina and the optional other carriers in the carrier.

According to the ammoniation reaction catalyst of the invention, preferably, the doping element doped in the carrier is from acid radical ions excluding chloride ions. Since the doped doping element is introduced during the preparation process of the carrier, the doped doping element is mainly in the bulk phase of the carrier.

According to the ammoniation reaction catalyst of the invention, preferably, the acid radical ion may be at least one selected from non-metallic acid radical ions, and further preferably at least one of borate ions, fluoride ions, phosphate ions, sulfate ions and selenate ions. **The** doping element is preferably selected from at least one of boron, fluorine, phosphorus, sulfur and selenium.

According to the ammoniation reaction catalyst of the invention, preferably, the pore volume of the carrier with a pore diameter less than 7.5 nm accounts for 5-17%, more preferably 5-10% of the pore volume of the carrier, the pore volume with a pore diameter greater than or equal to 7.5 nm and less than 9 nm accounts for 5-17% of the pore volume of the carrier, the pore volume with a pore diameter greater than or equal to 9 nm and less than or equal to 27 nm accounts for 61-89.5% of the pore volume of the carrier, and the pore volume with a pore diameter greater than 27 nm accounts for 0.5-5%, more preferably 0.5-3% of the pore volume of the carrier. **The** inventors of the invention have found that a catalyst having a pore structure satisfying this preferred embodiment has more excellent catalytic performance.

According to the ammoniation reaction catalyst of the invention, preferably, the ammonia adsorption capacity of the carrier is preferably 0.3-0.5 mmol/g.

According to the ammoniation reaction catalyst of the invention, preferably, the L acid of the carrier accounts for 92-100%, preferably 96-100% of the sum of the L acid and the B acid. The proportion of L acid is measured by pyridine probe adsorption spectroscopy.

According to the ammoniation reaction catalyst of the invention, preferably, the specific surface area of the carrier is 105-220 m²/g, and the pore volume of the carrier is 0.4-1.1 ml/g.

According to the ammoniation reaction catalyst of the invention, preferably, the content of the active component may be 5-42 g, preferably 10-35 g per 100 g of the carrier in terms of components other than the doping element. According to the invention, in order to better give play to the performance of the catalyst of the invention, optimize the ratio of reaction products, and reduce undesired side reactions, the catalyst may further comprise an auxiliary agent. The auxiliary agent may be at least one selected from Group VIB, Group VIIB, Group IB, Group IIB and lanthanide elements, preferably at least one of Cr, Mo, W, Mn, Re, Cu, Ag, Au, Zn, La and Ce.

According to the ammoniation reaction catalyst of the invention, preferably, the content of the auxiliary agent may be 0-10 g, preferably 0.5-6 g per 100 g of the carrier in terms of components other than the doping element.

The reactor used in the ammoniation reaction is not limited in the invention, and may be any reactor capable of performing gas-liquid-solid reaction. Considering sufficiently reacting the reaction raw materials, improving the reaction efficiency and enhancing the reaction effect, preferably, the reactor used in the ammoniation reaction is one of a fixed bed, an autoclave, a trickle bed, and a fluidized bed, or may be other reactors that may ensure stable operation of the reaction.

According to the invention, in the method for preparing hexamethylenediamine, step (2) further comprises: separating the ammoniation reaction product to obtain a stream containing hexamethyleneimine and water. Optionally, the stream containing hexamethyleneimine and water is separated to obtain a stream containing hexamethyleneimine, and at least part of the stream containing hexamethyleneimine is returned to step (1).

In order to further improve the yield of hexamethylenediamine, the stream containing hexamethyleneimine and water may be separated to obtain a stream containing hexamethyleneimine, and at least part of the stream containing hexamethyleneimine is returned to step (1). According to the invention, from an overall consideration of energy consumption of the system, the yield of hexamethylenediamine and the service life of the catalyst, preferably, the weight ratio of the stream containing hexamethyleneimine returned to step (1) to the fresh hexanediol is 0.1-26:1, more preferably 0.3-24:1, such as 0.5-20:1, 1-15:1, 2-10:1, and 3-8:1.

According to the invention, in the method for preparing hexamethylenediamine, step (2) further comprises: separating the ammoniation reaction product to obtain a stream containing aminohexanol. Optionally, at least part of the stream containing aminohexanol is returned to step (1).

In order to further improve the yield of hexamethylenediamine, at least part of the stream containing aminohexanol may be returned to step (1). According to the invention, from an overall consideration of energy consumption of the system, the yield of hexamethylenediamine and the service life of the catalyst, preferably, the weight ratio of the stream containing aminohexanol returned to step (1) to the fresh hexanediol is 0.1-26:1, more preferably 0.3-24:1, such as 0.5-20:1, 1-15:1, 2-10:1, and 3-8:1. The stream containing aminohexanol may also contain 0-30 wt% of hexanediol, 0-2 wt% of hexamethylenediamine, and small amounts of other components (e.g., heavy components).

It should be noted that, at the beginning of the reaction, the amount of hexamethyleneimine generated by the ammoniation reaction is small and cannot meet the above-defined weight ratio of the stream containing hexamethyleneimine to the fresh hexanediol. Thus in the initial stage of the reaction, all the stream containing hexamethyleneimine is returned to the ammoniation reaction. During the stable operation stage of the reaction, the amount of hexamethyleneimine accumulatively generated by the ammoniation reaction is sufficiently high, and at this time, it is only necessary to ensure that the returned stream containing hexamethyleneimine meets the above-defined weight ratio. **The** same is true for the aminohexanol generated by the ammoniation reaction. **The** weight ratio of the stream returned to step (1) to the fresh hexanediol given in the examples of the description is the mass ratio in the stable operation stage of the reaction.

According to the invention, in the method for preparing hexamethylenediamine, step (2) further comprises: separating the ammoniation reaction product to obtain a stream containing hexamethyleneimine and water, separating the stream containing hexamethyleneimine and water to obtain a stream containing hexamethyleneimine, and returning at least part of the stream containing hexamethyleneimine to step (1); and separating the ammoniation reaction product to obtain a stream containing aminohexanol, and returning at least part of the stream containing aminohexanol to step (1). Thereby, the yield of hexamethylenediamine may be further increased.

According to the invention, from an overall consideration of energy consumption of the system, the yield of hexamethylenediamine and the service life of the catalyst, preferably, the weight ratio of the stream containing hexamethyleneimine and the stream containing aminohexanol returned to step (1) is 0.1-5:1, such as 0.2-4.5:1, 0.3-4:1, 0.4-3.5:1, 0.5-3:1, 0.6-2.5:1, 0.7-2:1, and 0.8-1.5:1. Preferably, the ratio of the total weight of the stream containing hexamethyleneimine and the stream containing aminohexanol returned to step (1) to the weight of the fresh hexanediol is 0.1-30:1, preferably 0.3-25:1, such as 0.4-20:1, 0.5-15:1, 0.8-10:1, 1-8:1, 1.5-6:1, and 2-5:1

It should be noted that since the amount of hexamethyleneimine and aminohexanol generated by the ammoniation reaction is small at the beginning of the reaction and cannot meet the above-defined ratio of the total weight of the stream containing hexamethyleneimine and the stream containing aminohexanol to the weight of the fresh hexanediol, the stream containing hexamethyleneimine and the stream containing aminohexanol are all returned to the ammoniation reaction in the initial stage of the reaction. During the stable operation stage of the reaction, the amount of hexamethyleneimine and aminohexanol accumulatively generated by the ammoniation reaction is relatively large, and at this time, it is only necessary to ensure that the returned stream containing hexamethyleneimine and stream containing aminohexanol meet the above-defined weight ratio. The weight ratio of the streams returned to step (1) to the fresh hexanediol given in the examples of the description is the mass ratio in the stable operation stage of the reaction.

According to the invention, preferably, the conditions for separating the ammoniation reaction product are such that the content of hexamethyleneimine in the stream containing hexamethyleneimine and water is 35-95wt%, more preferably 40-85wt%, most preferably 50-80wt%; and the water content is 5-65wt%, more preferably 20-50wt%, most preferably 30-40wt%.

Preferably, the approach for separating the ammoniation reaction product is as follows: subjecting the ammoniation reaction product to a first separation to obtain a stream containing hexamethyleneimine and water, followed by a second separation to obtain a hexamethylenediamine product and a stream containing aminohexanol; more preferably, the first separation and the second separation each independently include at least one of distillation, membrane separation and pressure swing adsorption.

Preferably, the approach for separating the ammoniation reaction product is as follows: subjecting the ammoniation reaction product to a first separation to obtain a stream containing hexamethyleneimine and water, followed by a second separation to obtain a hexamethylenediamine product, and a stream containing aminohexanol and heavy components, and followed by a third separation to obtain a stream containing aminohexanol, and the heavy components; more preferably, the first separation, the second separation and the third separation each independently include at least one of distillation, membrane separation and pressure swing adsorption.

In the invention, for example, when distillation is used, the conditions for the first separation may include: a theoretical plate number of 10-80 (e.g., 10, 20, 30, 40, 50, 60, 70, 80, and a range constituted by any point values above), an operating pressure of 1-600 KPa (absolute pressure, e.g., 1 KPa, 10 KPa, 20 KPa, 30 KPa, 40 KPa, 50 KPa, 100 KPa, 200 KPa, 300 KPa, 400 KPa, 500 KPa, 600 KPa, and a range constituted by any point values above), a column bottom temperature of 90-300°C, and a reflux ratio of 0.1-15; the conditions for the second separation may include: a theoretical plate number of 10-80 (e.g., 10, 20, 30, 40, 50, 60, 70, 80, and a range constituted by any point values above), an operating pressure of 1-600 KPa (absolute pressure, for example, 1KPa, 10 KPa, 20 KPa, 30 KPa, 40 KPa, 50 KPa, 100 KPa, 200 Kpa, 300 KPa, 400 KPa, 500 KPa, 600 KPa, and a range constituted by any point values above), a column bottom temperature of 130-380°C, and a reflux ratio of 0.1-40; and the conditions for the third separation may include: a theoretical plate number of 10-100 (e.g., 10, 20, 30, 40, 50, 60, 70, 80, 90, 100, and a range constituted by any point values above), an operating pressure of 1-1100 KPa (absolute pressure, e.g., 1 KPa, 10 KPa, 20 KPa, 30 KPa, 40 KPa, 50 KPa, 100 KPa, 200 KPa, 300 KPa, 400 KPa, 500 KPa, 600 KPa, 700 KPa, 800 KPa, 900 KPa, 1000 KPa, 1100 KPa, and a range constituted by any point values above), a column bottom temperature of 150-400°C, and a reflux ratio of 0.1-70. The separation conditions of the invention may make the purity of the hexamethylenediamine product above 99.7wt%.

More preferably, the approach for separating the ammoniation reaction product is as follows: the ammoniation reaction product enters a first distillation column for separation, and a stream containing hexamethyleneimine and water is obtained at the top of the first distillation column; the bottom stream of the first distillation column is sent to a second distillation column for separation, a hexamethylenediamine product is obtained at the top of the column, and a stream containing hexanediol, aminohexanol and heavy components is obtained at the bottom of the column; the bottom stream of the second distillation column is sent to a third distillation column for separation, a stream containing hexanediol and aminohexanol is obtained at the top of the third distillation column, and heavy components are obtained at the bottom of the column.

According to the invention, preferably, the conditions for operating the first distillation column include: a column bottom temperature of 90-300°C, a column top temperature of 30-65°C, a reflux ratio of 0.1-10, a column top operating pressure of -0.1 MPaG to 0.5 MPaG, and a theoretical plate number of 10-30.

According to the invention, preferably, the conditions for operating the second distillation column include: a column bottom temperature of 120-320°C, a column top temperature of 40-85°C, a reflux ratio of 0.5-30, a column top operating pressure of -0.1 MPaG to 0.5 MPaG, and a theoretical plate number of 10-45.

According to the invention, preferably, the conditions for operating the third distillation column include: a column bottom temperature of 150-360°C, a column top temperature of 40-85°C, a reflux ratio of 1-65, a column top operating pressure of -0.1MPaG to 0MPaG, and a theoretical plate number of 15-85.

According to the invention, the approach for separating the stream containing hexamethyleneimine and water may be a dehydration commonly used in the art, but in order to further reduce the water content in the stream containing hexamethyleneimine, preferably, the dehydration includes at least one of atmospheric distillation, vacuum distillation, pressure swing distillation, azeotropic distillation, membrane separation, extractive distillation, adsorption dehydration, biological treatment dehydration and centrifugal separation. **The** azeotropic agent used in the azeotropic distillation may be at least one of cyclohexane, n-hexane, trimethylpentane, 1-methyl-4-isopropylbenzene, 1,4-dioxane, phenol, cresol, dibutyl ether, diamyl ether and diisoamyl ether. **The** conditions for operating the azeotropic distillation are specifically selected according to the different azeotropic points formed by the specific azeotropic agent, water and hexamethyleneimine.

Preferably, the water content in the stream containing hexamethyleneimine returned to step (1) is less than 3 wt%, preferably less than 1.5 wt%, more preferably less than 1 wt%.

According to a preferred embodiment of the invention, the dehydration for the stream containing hexamethyleneimine and water may be membrane separation dehydration, and the conditions for operating the membrane separation dehydration include: a membrane inlet pressure of 0-5MPaG, such as 1-4MPaG, and a temperature of 10-350°C, such as 10-200°C, such as 15-190°C.

According to a preferred embodiment of the invention, the dehydration for the stream containing hexamethyleneimine and water may be azeotropic distillation dehydration, and the azeotropic distillation dehydration is carried out in a distillation column. **The** conditions for operating the distillation column include: a weight ratio of the azeotropic agent to the stream containing hexamethyleneimine of 10-100:1, a column bottom temperature of 120-200°C, a column top temperature of 80-150°C, a reflux ratio of 0.1-20, a column top operating pressure of 0-3MPaG, and a plate number of 10-80.

According to a preferred embodiment of the invention, the dehydration for the stream containing hexamethyleneimine and water may be carried out by using distillation and membrane separation or pressure swing distillation at the same time. For example, the stream containing hexamethyleneimine and water is distilled in a distillation column, a stream containing hexamethyleneimine is obtained at the bottom of the column, and the stream at the top of the column is subjected to membrane separation or pressure swing distillation to obtain waste water. Preferably, the conditions for distilling the stream containing hexamethyleneimine and water include: a column bottom temperature of 60-250°C, and a column top operating pressure of -0.09 MPaG to 1 MPaG. Preferably, the membrane separation conditions include a membrane inlet temperature of 80-350°C and a membrane inlet pressure of 0-5 MPaG. The pressure swing operation range of the pressure swing distillation may be 0-8MPaG, preferably 0-5MPaG; more preferably, the pressure swing distillation is carried out in a high-pressure column and a low-pressure column, the column top pressure is 1-8MPaG and the column bottom temperature is 150-240°C in the high-pressure column, and the column top pressure is less than 1MPaG and the column bottom temperature is 50-130°C in the low-pressure column.

According to the invention, preferably, before separating the stream containing hexamethyleneimine and water, the hexamethylenediamine product, the stream containing aminohexanol and the heavy components from the ammoniation reaction product, the ammoniation reaction product is first pre-separated to recover hydrogen and ammonia in the ammoniation reaction product; wherein the recovered hydrogen and ammonia are first condensed to obtain circulating ammonia (liquid phase), and then compressed to obtain circulating hydrogen (gas phase), and the circulating hydrogen and the circulating ammonia are returned to the ammoniation reaction. Preferably, the pre-separation includes at least one of flash evaporation, distillation and stripping. The pre-separation method may also employ other methods capable of recovering circulating hydrogen and ammonia from the ammoniation reaction product. More preferably, in the circulating hydrogen, the content of hydrogen is 5-40 wt%, and the content of ammonia is 60-95 wt%; in the circulating ammonia, the content of ammonia is 40-100 wt%, the content of water is 0-1 wt%, the content of hexamethylenediamine is 0-1 wt%, and the content of hexamethyleneimine is 0-1 wt%. Since a part of ammonia and hydrogen will be consumed during the ammoniation reaction, and a small amount of hydrogen and ammonia will also be lost during the hydrogen and ammonia recovery process, it is necessary to supplement fresh ammonia and hydrogen during the ammoniation reaction to maintain the molar ratio of hexanediol (including freshly supplemented hexanediol and hexanediol in the circulating stream), ammonia and hydrogen in the ammoniation reaction system.

To ensure the effective utilization of hydrogen and ammonia and reduce the overall energy consumption of the process, preferably, the pre-separation conditions are such that the recovery rate of hydrogen is greater than 99% and the recovery rate of ammonia is greater than 98%. In the invention, the pre-separation may adopt a multi-stage vacuum flash evaporation, the multi-stage flash evaporation may include at least two stages of flash evaporation, the pressure of the flash evaporation decreases with a gradient of 1-8 MPa, and the pressure of the last stage of the flash evaporation is 0.5-1 MPaG. For example, a four-stage flash evaporation is adopted, and the flash evaporation pressures of the first stage to the fourth stage are 7-15 MPaG, 4-7 MPaG, 1-4 MPaG, and 0.1-1 MPaG, respectively. For example, a three-stage flash evaporation is adopted, and the flash evaporation pressures of the first stage to the third stage are 7-15 MPaG, 1-7 MPaG, and 0.5-3 MPaG, respectively.

According to the invention, in the method for preparing hexamethylenediamine, step (4) further comprises: before the ammonolysis reaction, dissolving the heavy components in a second solvent, and then carrying out the ammonolysis. The second solvent is a solvent in which the solubility of the ammoniation reaction raw materials and reaction products such as hexanediol, hexamethylenediamine, aminohexanol, hexamethyleneimine, and dihexamethylenetriamine is greater than 0.05 g/g, for example, at least one of tetrahydrofuran, 1,4-dioxane, n-hexane, cyclohexane, tert-butanol, etc. The weight ratio of the second solvent to the heavy components is 1-20:1, preferably 2-18:1, and more preferably 3-15:1.

The second solvent for dissolving the heavy components may be of the same type as the first solvent used in the ammoniation reaction (if a solvent is present). The solvent for dissolving the heavy components may be a freshly introduced solvent or a solvent stream separated from the ammoniation reaction product (if a solvent is present in the ammoniation reaction). Usually, in the initial stage of the reaction, the amount of solvent in the system is small, and a fresh solvent may be used to dissolve the heavy components at this time. After the reaction runs for a period of time, the amount of solvent in the system is relatively large, and the solvent stream separated from the ammoniation reaction product may meet the amount of solvent required for the ammoniation reaction and the ammonolysis reaction. At this time, no fresh solvent is introduced to the system, and a part of the solvent stream separated from the ammoniation reaction product is returned to the ammoniation reaction, and the other part is used to dissolve the heavy components. Since the solvent circulating in the system will inevitably be lost during running of the reaction, a fresh solvent is regularly supplemented to the system.

According to the invention, in step (3), the ammonolysis is carried out in the presence of hydrogen and ammonia, and the molar ratio of hydrogen:ammonia:heavy components is 0.1-20:10-150:1, preferably 0.2-15:15-120:1, more preferably 0.2-10:20-100:1, and most preferably 0.3-8:30-90:1, wherein the heavy components are calculated as dihexamethylenetriamine.

According to the invention, preferably, the conditions of the ammonolysis reaction include: a reaction temperature of 120-300°C, preferably 150-270°C; a reaction pressure of 9-25MPaG, preferably 10-22MPaG; and a liquid volume space velocity of the heavy components of 0.01-8h⁻¹, preferably 0.05-5h⁻¹.

**In** the invention, the ammonolysis catalyst used in the ammonolysis reaction may be prepared according to the following method:
(1) preparing a carrier: contacting a mixture of pseudo-boehmite, silica sol and calcium nitrate with an aqueous solution containing nitric acid and phosphoric acid, followed by being kneaded, dried and calcined in sequence;
(2) impregnating the carrier in an aqueous solution containing nickel sulfate, lanthanum acetate and indium nitrate, drying them at 100-140°C for 2-6 hours, followed by being calcined at 350-450°C. for 2-6 hours.

According to the method for preparing the ammonolysis catalyst of the invention, preferably, in step (1), the amount of silica sol is 0.6-0.8 g, the amount of calcium nitrate is 0.1-0.4 g, and the aqueous solution containing nitric acid and phosphoric acid is 0.2-0.5 g per gram of pseudo-boehmite.

According to the method for preparing the ammonolysis catalyst of the invention, preferably, in step (1), in the aqueous solution containing nitric acid and phosphoric acid, the content of nitric acid is 10-25 wt%, and the content of phosphoric acid is 5-15 wt%.

According to the method for preparing the ammonolysis catalyst of the invention, preferably, in step (1), the drying temperature is 100-140°C, and the drying time is 1-6 hours.

According to the method for preparing the ammonolysis catalyst of the invention, preferably, in step (2), the amount of nickel sulfate is 0.6-0.85 g, the amount of lanthanum acetate is 0.06-0.08 g, and the amount of indium nitrate is 0.055-0.065 g per gram of the carrier.

According to the method for preparing the ammonolysis catalyst of the invention, preferably, in step (2), in the aqueous solution containing nickel sulfate, lanthanum acetate and indium nitrate, the concentration of nickel sulfate is 20-25 wt%, the concentration of lanthanum acetate is 1.5-2.5 wt%, and the concentration of indium nitrate is 1.5-2.5 wt%. **The** impregnation method is preferably an isovolumetric impregnation method, and the impregnation may be performed in multiple times.

**The** invention also finds that, by returning the stream containing aminohexanol and the stream containing hexamethyleneimine to the ammoniation reaction in a certain proportion and subjecting the heavy components from the ammoniation reaction to ammonolysis, not only the yield of hexamethylenediamine is greatly improved, but also the discharge amount of the ammoniation reaction waste liquid is reduced, thereby greatly reducing the cost of producing hexamethylenediamine.

### The presence of the first solvent in the ammoniation reaction

**In** the method for preparing hexamethylenediamine of the invention,
step (1) comprises: under an ammoniation reaction condition, subjecting hexanediol and ammonia to an ammoniation reaction in the first solvent to obtain an ammoniation reaction product; and
step (2) comprises: separating the ammoniation reaction product from step (1) to obtain a stream containing hexamethyleneimine, the first solvent and water, a hexamethylenediamine product, a stream containing aminohexanol, and heavy components.

**In** an embodiment of the invention, the method for preparing hexamethylenediamine comprises the following steps:
(1) under an ammoniation reaction condition, subjecting hexanediol and ammonia to an ammoniation reaction in the first solvent to obtain an ammoniation reaction product;
(2) separating the ammoniation reaction product from step (1) to obtain a stream containing hexamethyleneimine, the first solvent and water, a hexamethylenediamine product, a stream containing aminohexanol, and heavy components; and
(3) under an ammonolysis reaction condition, subjecting the heavy components to ammonolysis to obtain an ammonolysis reaction product, and returning the ammonolysis reaction product to step (2).

**In** an embodiment of the invention, the method for preparing hexamethylenediamine comprises the following steps:
(1) under an ammoniation reaction condition, subjecting hexanediol and ammonia to an ammoniation reaction in the first solvent to obtain an ammoniation reaction product;
(2) separating the ammoniation reaction product from step (1) to obtain a stream containing hexamethyleneimine, the first solvent and water, a hexamethylenediamine product, a stream containing aminohexanol, and heavy components, then separating the stream containing hexamethyleneimine, the first solvent and water to obtain a stream containing hexamethyleneimine and a first solvent stream, and returning at least part of the stream containing hexamethyleneimine and at least part of the first solvent stream to step (1); and
(3) under an ammonolysis reaction condition, subjecting the heavy component to ammonolysis to obtain an ammonolysis reaction product, and returning the ammonolysis reaction product to step (2).

**In** an embodiment of the invention, the method for preparing hexamethylenediamine comprises the following steps:
(1) under an ammoniation reaction condition, subjecting hexanediol and ammonia to an ammoniation reaction in the first solvent to obtain an ammoniation reaction product;
(2) separating the ammoniation reaction product from step (1) to obtain a stream containing hexamethyleneimine, the first solvent and water, a hexamethylenediamine product, a stream containing aminohexanol, and heavy components, returning at least part of the stream containing aminohexanol to step (1), then separating the stream containing hexamethyleneimine, the first solvent and water to obtain a stream containing hexamethyleneimine and a first solvent stream, and returning at least part of the stream containing hexamethyleneimine and at least part of the first solvent stream to step (1); and
(3) under an ammonolysis reaction condition, subjecting the heavy components to ammonolysis to obtain an ammonolysis reaction product, and returning the ammonolysis reaction product, with or without separation, to step (2).

Introducing the solvent into the ammoniation reaction may maintain a uniform temperature distribution in the system, which is beneficial to reducing reaction hot spots, helps to improve reaction stability, and ensures that no solid phase crystallization and separation occur throughout the system, reducing the operating temperature of each equipment in the device, and avoiding the huge investment brought by device insulation. **In** addition, introducing the solvent may effectively dissolve hexanediol, and by-products such as hexamethyleneimine and aminohexanol produced by the ammoniation reaction, which helps to solve the problem of pipeline blockage during the entire operation cycle, especially in the separation process.

**In** the invention, the first solvent is introduced at a one-time time (with hexanediol in the initial stage of the reaction). **In** the initial stage of the reaction (before returning the circulating stream to the ammoniation reaction), the molar ratio of the first solvent to hexanediol is 0.1-10:1, preferably 0.15-10:1, more preferably 0.25-9:1. However, it is understandable that the first solvent will be lost during the circulation and purification process. **In** order to ensure the amount of circulating solvent in the system, a small amount of fresh solvent may be supplemented during the first solvent circulation process to maintain a constant amount of the solvent in the system. According to the invention, the first solvent is a solvent in which the solubility of the ammoniation reaction raw materials and reaction products such as hexanediol, hexamethylenediamine, aminohexanol and hexamethyleneimine is greater than 0.1g/g, such as at least one of tetrahydrofuran, 1,4-dioxane, n-hexane, cyclohexane, tert-butanol, tetramethylene sulfone, glycerol, etc.

Preferably, in step (1), during the ammoniation reaction, the molar ratio of ammonia to hexanediol is 10-80:1, preferably 20-45:1; the ammoniation reaction is carried out in the presence of hydrogen, and the molar ratio of hydrogen to hexanediol is 0.05-20:1, preferably 0.2-5:1. **The** molar ratio of ammonia, hydrogen and hexanediol refers to the molar ratio in the mixture at the inlet of the ammoniation reactor.

Preferably, in step (1), the temperature of the ammoniation reaction is 100-250°C, preferably 130-210°C; the pressure of the ammoniation reaction is 6-18 MPaG, preferably 8-16 MPaG, and the liquid volume space velocity of the fresh hexanediol is 0.01-10h⁻¹, preferably 0.05-4h⁻¹.

Preferably, the weight ratio of the stream containing hexamethyleneimine and the stream containing aminohexanol returned to step (1) is 0.1-5:1, for example, 0.2-4.5:1, 0.3-4:1, 0.4-3.5:1, 0.5-3:1, 0.6-2.5:1, 0.7-2:1, and 0.8-1.5:1, so as to further improve the yield of hexamethylenediamine. Since the solvent only serves to dissolve the reaction raw material and reaction product, there is no restriction on the amount of the returned solvent, and it may be all returned or partially returned. When a part of the solvent is returned to step (1), for example, the weight ratio of the stream containing hexamethyleneimine, the first solvent stream and the stream containing aminohexanol may be 0.1-5:0.3-40:1, preferably 0.1-2.5:0.3-10:1, and more preferably 0.5-2:1-10:1.

Preferably, the ratio of the total weight of the stream containing hexamethyleneimine and the stream containing aminohexanol returned to step (1) to the weight of the fresh hexanediol is 0.1-30:1, preferably 0.3-25:1, for example, 0.4-20:1, 0.5-15:1, 0.8-10:1, 1-8:1, 1.5-6:1, and 2-5:1, so as to further improve the yield of hexamethylenediamine.

Preferably, step (2) further comprises: before separating the stream containing hexamethyleneimine, the first solvent and water, the hexamethylenediamine product, the stream containing aminohexanol, and the heavy components from the ammoniation reaction product, recovering hydrogen and ammonia in the ammoniation reaction product; wherein the recovered hydrogen and ammonia are first condensed to obtain circulating ammonia (liquid phase), and then compressed to obtain circulating hydrogen (gas phase), and the circulating hydrogen and circulating ammonia are returned to the ammoniation reaction. More preferably, in the circulating hydrogen, the content of hydrogen is 5-40 wt%, and the content of ammonia is 60-95 wt%; in the circulating ammonia, the content of ammonia is 40-100 wt%, the content of the solvent is 0-60 wt%, the content of water is 0-1 wt%, the content of hexamethylenediamine is 0-1 wt%, and the content of hexamethyleneimine is 0-1 wt%. Since a part of ammonia and hydrogen will be consumed during the ammoniation reaction, and a small amount of hydrogen and ammonia will also be lost during the hydrogen and ammonia recovery process, it is necessary to supplement fresh ammonia and hydrogen during the ammoniation reaction to maintain the molar ratio of hexanediol (including freshly supplemented hexanediol and hexanediol in the circulating stream), ammonia and hydrogen in the ammoniation reaction system.

Preferably, step (2) comprises: feeding the ammoniation reaction product from which ammonia and hydrogen are optionally removed, into a first distillation column for separation, to obtain a stream containing hexamethyleneimine, water and the first solvent at the top of the first distillation column; feeding the bottom stream of the first distillation column into a second distillation column for separation, to obtain a hexamethylenediamine product at the top of the column, and a stream containing hexanediol, aminohexanol and heavy components at the bottom of the column; and feeding the bottom stream of the second distillation column into a third distillation column for separation, to obtain a stream containing hexanediol and aminohexanol at the top of the third distillation column, and the heavy components at the bottom of the column.

Preferably, the conditions for operating the first distillation column include: a column bottom temperature of 90-300°C, a column top temperature of 30-65°C, a reflux ratio of 0.1-10, a column top operating pressure of -0.1 MPaG to 0.5 MPaG, and a plate number of 10-30.

Preferably, the conditions for operating the second distillation column include: a column bottom temperature of 120-320°C, a column top temperature of 40-85°C, a reflux ratio of 0.5-30, a column top operating pressure of -0.1 MPaG to 0.5 MPaG, and a plate number of 10-45.

Preferably, the conditions for operating the third distillation column include: a column bottom temperature of 150-360°C, a column top temperature of 40-85°C, a reflux ratio of 1-65, a column top operating pressure of -0.1 MPaG to 0 MPaG, and a plate number of 15-85.

Preferably, step (2) comprises: distilling the stream containing hexamethyleneimine, the first solvent and water in a distillation column, to obtain a stream containing hexamethyleneimine at the bottom of the column, and subjecting the stream at the top of the column to membrane separation or pressure swing distillation to obtain a solvent stream and waste water. Preferably, the conditions for distilling the stream containing hexamethyleneimine, the first solvent and water include: a column bottom temperature of 60-250°C, and a column top operating pressure of -0.09 MPaG to 1 MPaG. Preferably, the membrane separation conditions include a membrane inlet temperature of 80-350°C and a membrane inlet pressure of 0-5 MPaG. The pressure swing operation range of the pressure swing distillation may be 0-8MPaG, preferably 0-5MPaG; more preferably, the pressure swing distillation is carried out in a high-pressure column and a low-pressure column, the column top pressure is 1-8MPaG and the column bottom temperature is 150-240°C in the high-pressure column; the column top pressure is less than 1MPaG and the column bottom temperature is 50-130°C in the low-pressure column.

Preferably, the ammonolysis reaction product is roughly separated by distillation, and the conditions in the rough fractionation column for rough separation include: a column bottom temperature of 380-520°C, a column top operating pressure of -0.05MPag to 1MPag, and a reflux ratio of 2-50.

### Absence of the first solvent in the ammoniation reaction

In the method for preparing hexamethylenediamine of the invention,
step (1) comprises: under an ammoniation reaction condition, and in the absence of the first solvent, subjecting hexanediol and ammonia to an ammoniation reaction to obtain an ammoniation reaction product.

In an embodiment of the invention, the method for preparing hexamethylenediamine comprises the following steps:
(1) under an ammoniation reaction condition, and in the absence of the first solvent, subjecting hexanediol and ammonia to an ammoniation reaction to obtain an ammoniation reaction product;
(2) separating the ammoniation reaction product from step (1) to obtain a stream containing hexamethyleneimine and water, a hexamethylenediamine product, a stream containing aminohexanol, and heavy components; and
(3) under an ammonolysis reaction condition, subjecting the heavy components to ammonolysis to obtain an ammonolysis reaction product, and returning the ammonolysis reaction product to step (2).

In an embodiment of the invention, the method for preparing hexamethylenediamine comprises the following steps:
(1) under an ammoniation reaction condition, and in the absence of the first solvent, subjecting hexanediol and ammonia to an ammoniation reaction to obtain an ammoniation reaction product;
(2) separating the ammoniation reaction product from step (1) to obtain a stream containing hexamethyleneimine and water, a hexamethylenediamine product, a stream containing aminohexanol, and heavy components, and then separating the stream containing hexamethyleneimine and water to obtain a stream containing hexamethyleneimine, and returning at least part of the stream containing hexamethyleneimine to step (1) ; and
(3) under an ammonolysis reaction condition, subjecting the heavy components to ammonolysis to obtain an ammonolysis reaction product, and returning the ammonolysis reaction product to step (2).

**In** an embodiment of the invention, the method for preparing hexamethylenediamine comprises the following steps:
(1) under an ammoniation reaction condition, and in the absence of the first solvent, subjecting hexanediol and ammonia to an ammoniation reaction to obtain an ammoniation reaction product;
(2) separating the ammoniation reaction product from step (1) to obtain a stream containing hexamethyleneimine and water, a hexamethylenediamine product, a stream containing aminohexanol, and heavy components, returning at least part of the stream containing aminohexanol to step (1), and then separating the stream containing hexamethyleneimine and water to obtain a stream containing hexamethyleneimine, and returning at least part of the stream containing hexamethyleneimine to step (1) ; and
(3) under an ammonolysis reaction condition, subjecting the heavy components to ammonolysis to obtain an ammonolysis reaction product, and returning the ammonolysis reaction product, with or without separation, to step (2).

Preferably, in step (1), the molar ratio of ammonia to hexanediol is 18-50:1, preferably 22-38:1, and the molar ratio of hydrogen to hexanediol is 0.08-6:1, preferably 0.4-3:1. **The** molar ratio of ammonia, hydrogen and hexanediol refers to the molar ratio in the mixture at the inlet of the ammoniation reactor.

Preferably, in step (1), the temperature of the ammoniation reaction is 120-210°C, preferably 130-200°C. **The** pressure of the ammoniation reaction is 7-17 MPaG, preferably 9-16 MPaG. The liquid volume space velocity of the fresh hexanediol is 0.05-7h⁻¹, preferably 0.09-3.9h⁻¹.

Preferably, the weight ratio of the stream containing hexamethyleneimine returned to step (1) to the fresh hexanediol is 0.1-13:1, preferably 0.3-10:1.

### Examples

The invention will be described in detail below through examples. In the following examples,
the composition of the products was analyzed by gas chromatography.

The yield of hexamethylenediamine = the molar amount of hexamethylenediamine ÷ the molar amount of freshly fed hexanediol × 100%.

### Preparation Example 1

An ammonolysis catalyst is prepared by a multi-step impregnation method:
(1) 94.2 g of pseudo-boehmite (produced by the aluminum sulfate method, with a specific surface area of 310 m²/g and a pore volume of 1.19 ml/g), 72.5 g of silica sol (JN-40), and 25.26 g of calcium nitrate tetrahydrate were weighed. The pseudo-boehmite was placed in a kneader, and the weighed silica sol and calcium nitrate tetrahydrate were added to 24.77g of water to prepare a solution, which was added to the kneader and thoroughly stirred with the pseudo-boehmite. Next, an aqueous solution prepared from 16.51g of water, 4.71g of nitric acid and 2.83g of phosphoric acid was added and stirred thoroughly. The mixture was then kneaded and extruded into a clover shape, dried at 120°C for 4 hours, and subsequently calcined in a muffle furnace at 900°C for 6 hours. After cooling, a carrier is obtained.
(2) 100.77 g of nickel sulfate hexahydrate (industrial grade, purity 98%), 5.69 g of lanthanum acetate monohydrate and 5.96 g of indium nitrate pentahydrate were added to 134.78 mL of water to prepare an aqueous solution,. The solution was loaded on 73.25 g of the carrier obtained in step (1) by an isovolumetric impregnation method in two times, and dried at 120°C for 4 hours after each impregnation. After the two impregnations, it was calcined at 390°C for 4 hours.

### Example 1

(1) Raw materials, i.e., a solvent (cyclohexane), hexanediol, ammonia and hydrogen, were fed into a fixed-bed ammoniation reactor loaded with a catalyst, which was the catalyst in Example 14 of CN114433086A. The molar ratio of ammonia to hexanediol was 35:1, the molar ratio of hydrogen to hexanediol was 0.5:1, the molar ratio of the solvent to hexanediol was 3.8:1, the ammoniation reaction temperature was 166°C, the ammoniation reaction pressure was 12 MPaG, and the liquid volume space velocity of fresh hexanediol was 0.1h⁻¹. The product of the initial ammoniation reaction (before the circulating stream was returned to the fixed-bed ammoniation reactor) comprised: 5.76 wt% of hexamethylenediamine, 1.13 wt% of hexanediol, 4.91 wt% of aminohexanol, 4.76 wt% of hexamethyleneimine, 48.1 wt% of ammonia, 31.59 wt% of cyclohexane, 2.56 wt% of water, and the remainder as heavy components. The molar yield of hexamethylenediamine in the initial ammoniation reaction was 31.1%.
(2) The ammoniation reaction product was subjected to gas-liquid separation to remove hydrogen and ammonia therein, and the hydrogen was returned to the fixed-bed ammoniation reactor in the form of gas phase via a compressor, and the liquid ammonia was pumped back to the ammoniation reactor. The gas-liquid separation method comprised: subjecting the reaction product to gas-liquid separation via four stages of flash tanks at 12MPaG, 5MPaG, 2MPaG and 0.5MPaG, wherein the gas phases at the top of the second-stage and third-stage flash tanks were respectively condensed and refluxed at a reflux ratio of 0.1, and the liquid phase was heated before entering the fourth-stage flash tank which was provided with a 1 meter of filler. The gas phase obtained by flash evaporation was condensed with condensed water at 30-45°C to obtain circulating ammonia (liquid phase) and a gas phase (hydrogen), and the gas phase was compressed to obtain circulating hydrogen. The circulating hydrogen mainly comprised 9.72 wt% of hydrogen and the remainder as ammonia; and the circulating ammonia mainly comprised 94.96 wt% of ammonia, 4.12 wt% of cyclohexane, 0.73 wt% of water, 0.01 wt% of hexamethylenediamine and 0.15 wt% of hexamethyleneimine.
   The ammoniation reaction product from which ammonia and hydrogen were removed entered a first distillation column for vacuum distillation, to obtain a stream containing hexamethyleneimine, water and solvent (the stream comprised: 80.11 wt% of cyclohexane, 13.01 wt% of hexamethyleneimine, and 5.79 wt% of water) at the top of the first distillation column; the conditions for operating the first distillation column included: a column bottom temperature of 179.9°C, a column top temperature of 40.5°C, a reflux ratio of 1.5, a column top operating pressure of -0.08MPa, and a plate number of 25. The bottom stream of the first distillation column was sent to a second distillation column for separation, to obtain a hexamethylenediamine product (the content of hexamethylenediamine in the product was 99.8 wt%) at the top of the column, and a stream containing hexanediol, aminohexanol and heavy components at the bottom of the column (the stream comprised 79.73 wt% of aminohexanol, 18.35 wt% of hexanediol, 0.2 wt% of hexamethylenediamine, and the remainder as the heavy components C12 amine); the conditions for operating the second distillation column included: a column bottom temperature of 189.1°C, a column top temperature of 83°C, a reflux ratio of 2.7, a column top operating pressure of -0.09MPa, and a plate number of 24. The bottom stream of the second distillation column was sent to a third distillation column for separation, to obtain a stream containing hexanediol and aminohexanol at the top of the third distillation column, and the heavy components at the bottom of the column; the conditions for operating the third distillation column included: a column bottom temperature of 267.5°C, a column top temperature of 83.7°C, a reflux ratio of 5.3, a column top operating pressure of -0.09MPa, and a plate number of 38.
(3) The stream containing hexamethyleneimine, water and solvent obtained in step (2) was distilled to obtain a stream containing hexamethyleneimine (the stream comprised 99.91 wt% of hexamethyleneimine and 0.09 wt% of hexamethylenediamine) and a stream containing solvent and water, wherein the distillation conditions included a column bottom temperature of 94.3°C. and a column top operating pressure of -0.08 MPa. Membrane separation was used to dehydrate the stream containing solvent and water to obtain a solvent stream (the stream comprised 99.67 wt% of cyclohexane and 0.32 wt% of hexamethyleneimine) and waste water (cyclohexane content was less than 100ppmw), and the conditions for membrane separation included a membrane inlet temperature of 100°C and a membrane inlet pressure of 0.05MPaG.
(4) The heavy components obtained at the bottom of the third distillation column in step (2) was mixed uniformly with the solvent (cyclohexane) and then sent to an ammonolysis reactor filled with the catalyst for ammonolysis, and ammonia and hydrogen were introduced into the ammonolysis reactor at the same time, wherein the molar ratio of hydrogen: ammonia: heavy components calculated as dihexamethylenetriamine was 1:40:1, the weight ratio of the solvent to the heavy components was 5:1, the ammonolysis reaction temperature was 186°C, the ammonolysis reaction pressure was 16 MPaG, and the liquid volume space velocity of the heavy components was 0.5h⁻¹.

The obtained ammonolysis reaction product had the composition: 0.07 wt% of hydrogen, 55.66 wt% of ammonia, 7.84 wt% of hexamethylenediamine, 6.01 wt% of hexamethyleneimine, 21.92 wt% of cyclohexane, and the remainder as the heavy components. The ammonolysis reaction product was all returned to step (2).

The ratio of the total weight of the stream containing hexamethyleneimine and the stream containing aminohexanol returned to step (1) to the weight of the fresh hexanediol was 2.88. The weight ratio of the stream containing hexamethyleneimine to the stream containing aminohexanol returned to step (1) was 0.72:1.

The system was continuously operated to a stable state, and the molar yield of hexamethylenediamine was 93.7%. The system had no blockage and could be operated continuously and smoothly for a long period of time.

In addition, the temperature distribution in the reactor containing the solvent was uniform without hot spots, and the temperature value deviation of multiple temperature measurement points distributed at the same bed height was less than 1.2°C.

### Example 2

(1) Raw materials, i.e., a solvent (1,4-dioxane), hexanediol, ammonia and hydrogen, were fed into a fixed-bed ammoniation reactor loaded with a catalyst, which was the catalyst in Example 6 in CN114433086A. The molar ratio of ammonia to hexanediol was 25:1, the molar ratio of hydrogen to hexanediol was 1:1, the molar ratio of the solvent to hexanediol was 1.2:1, the ammoniation reaction temperature was 190°C., the ammoniation reaction pressure was 10 MPaG, and the liquid volume space velocity of fresh hexanediol was 0.6h⁻¹. The product of the initial ammoniation reaction (before the circulating stream was returned to the fixed-bed ammoniation reactor) comprised: 8.39 wt% of hexamethylenediamine, 0.93 wt% of hexanediol, 5.97 wt% of aminohexanol, 6.67 wt% of hexamethyleneimine, 34.83 wt% of ammonia, 39.02 wt% of 1,4-dioxane, 3.12 wt% of water, and the remainder as heavy components. The molar yield of hexamethylenediamine in the initial ammoniation reaction was 36.4%.
(2) The ammoniation reaction product was subjected to gas-liquid separation to remove hydrogen and ammonia therein, and the hydrogen was returned to the fixed-bed ammoniation reactor in the form of gas phase via a compressor, and the liquid ammonia was pumped back to the ammoniation reactor. **The** gas-liquid separation method comprised: subjecting the reaction product to three-stage gas-liquid separation at 10MPaG, 2MPaG and 0.8MPaG, wherein a section of filler was provided at the top of the second-stage flash tank, and the gas phase at the top of the tank was condensed and refluxed at a reflux ratio of 0.3, and a 1 meter of filler and a tank bottom heater were provided in the third-stage flash tank. **The** gas phase obtained by flash evaporation was condensed with condensed water at 30-45°C to obtain circulating ammonia (liquid phase) and a gas phase (hydrogen), and the gas phase was compressed to obtain circulating hydrogen. **The** circulating hydrogen mainly comprised 10.19 wt% of hydrogen and the remainder as ammonia; and the circulating ammonia mainly comprised 93 wt% of ammonia, 5.79 wt% of 1,4-dioxane, 0.91 wt% of water, 0.01 wt% of hexamethylenediamine and 0.21 wt% of hexamethyleneimine.
   The ammoniation reaction product from which ammonia and hydrogen were removed entered a first distillation column for vacuum distillation, to obtain a stream containing hexamethyleneimine, water and the solvent (the stream comprised: 79.93 wt% of 1,4-dioxane, 14.40 wt% of hexamethyleneimine, and 5.86 wt% of water) at the top of the first distillation column; the conditions for operating the first distillation column included: a column bottom temperature of 176.9°C, a column top temperature of 44.2°C, a reflux ratio of 2.6, a column top operating pressure of -0.08MPa, and a plate number of 19. **The** bottom stream of the first distillation column was sent to a second distillation column for separation, to obtain a hexamethylenediamine product (the content of hexamethylenediamine in the product was 99.9 wt%) at the top of the column, and a stream containing hexanediol, aminohexanol and heavy components at the bottom of the column (the stream comprised 85.36 wt% of aminohexanol, 13.33 wt% of hexanediol, 0.25 wt% of hexamethylenediamine, and the remainder as the heavy components C12 amine); the conditions for operating the second distillation column included: a column bottom temperature of 188.3°C, a column top temperature of 84.3°C, a reflux ratio of 1.9, a column top operating pressure of -0.09MPa, and a plate number of 33. **The** bottom stream of the second distillation column was sent to a third distillation column for separation, to obtain a stream containing hexanediol and aminohexanol at the top of the third distillation column, and the heavy components at the bottom of the column; the conditions for operating the third distillation column included: a column bottom temperature of 270.5°C, a column top temperature of 83.6°C, a reflux ratio of 5, a column top operating pressure of -0.09MPa, and a plate number of 39.
(3) The stream containing hexamethyleneimine, water and the solvent obtained in step (2) was distilled to obtain a stream containing hexamethyleneimine (the stream comprised 99.94 wt% of hexamethyleneimine and 0.06 wt% of hexamethylenediamine) and a stream containing the solvent and water, wherein the distillation conditions included a column bottom temperature of 207.3°C and a column top operating pressure of 0.5MPa. Pressure swing distillation was used to dehydrate the stream containing the solvent and water to obtain a solvent stream (the stream comprised: 99.64 wt% of 1,4-dioxane and 0.36 wt% of hexamethyleneimine) and waste water (99.95 wt% of water and 0.05 wt% of 1,4-dioxane), and the pressure swing distillation conditions included a top pressure of 1MPaG and a bottom temperature of 197.2°C in a high-pressure column, and a top pressure of 0MPaG and a bottom temperature of 96.3°C in a low-pressure column.
(4) The heavy components obtained at the bottom of the third distillation column in step (2) was mixed uniformly with the solvent (1,4-dioxane) and then sent to an ammonolysis reactor filled with the catalyst for ammonolysis, and ammonia and hydrogen were introduced into the ammonolysis reactor at the same time, wherein the molar ratio of hydrogen: ammonia: heavy components calculated as dihexamethylenetriamine was 3:50:1, the weight ratio of the solvent to the heavy components was 11:1, the ammonolysis reaction temperature was 203°C, the ammonolysis reaction pressure was 15 MPaG, and the liquid volume space velocity of the heavy components was 0.8h⁻¹. The obtained ammonolysis reaction product had the composition: 4.75 wt% of hydrogen, 71.74 wt% of ammonia, 2.60 wt% of hexamethylenediamine, 3.52 wt% of hexamethyleneimine, 15.26 wt% of 1,4-dioxane, and the remainder as the heavy components. The ammonolysis reaction product was depressurized to 10 MPag and subjected to gas-liquid separation via flash evaporation at this pressure to obtain a stream containing hydrogen and ammonia and a residual liquid phase. The residual liquid phase was then subjected to distillation in a rough fractionation column to obtain a mixed liquid containing hexamethylenediamine and hexamethyleneimine, along with heavy components. The column top pressure was -0.07 MPag, the column top temperature was 180°C, the column bottom temperature was 480°C, and the reflux ratio was 20 in the rough fractionation column. The mixed liquid containing hexamethylenediamine and hexamethyleneimine at the top of the rough fractionation column was returned to step (2), and the bottom stream was discharged as waste liquid.

The ratio of the total weight of the stream containing hexamethyleneimine and the stream containing aminohexanol returned to step (1) to the weight of the fresh hexanediol was 2.95. The weight ratio of the stream containing hexamethyleneimine to the stream containing aminohexanol returned to step (1) was 0.97:1.

The system was continuously operated to a stable state, and the molar yield of hexamethylenediamine was 94.8%. The system had no blockage and could be operated continuously and smoothly for a long period of time.

In addition, the temperature distribution in the reactor containing the solvent was uniform without hot spots, and the temperature value deviation of multiple temperature measurement points distributed at the same bed height was less than 1.5°C.

### Example 3

(1) Ammonia, hydrogen and hexanediol were fed into a fixed-bed ammoniation reactor filled with a catalyst, which was the catalyst in Example 2 of CN114433113A, wherein the molar ratio of ammonia to hexanediol was 35:1, the molar ratio of hydrogen to hexanediol was 2:1, the ammoniation reaction temperature was 150°C, the reaction pressure was 12 MPa, and the liquid phase volume space velocity of hexanediol was 1h⁻¹.
(2) The ammoniation reaction product was subjected to gas-liquid separation to remove hydrogen and ammonia therein, and the hydrogen was returned to the fixed-bed ammoniation reactor in the form of gas phase via a compressor, and the liquid ammonia was pumped back to the ammoniation reactor. The gas-liquid separation method comprised: passing the ammoniation reaction product through three flash tanks in sequence for three-stage vacuum flash evaporation to recover hydrogen, wherein the pressures of the first flash tank to the third flash tank were set at 7MPa, 5MPa, and 2MPa, respectively, the gas phases at the top of the three flash tanks were cooled to 45°C respectively and then further subjected to gas-liquid separation, wherein the obtained liquid phase was returned to the respective vacuum flash tank, and the gas phase was pressurized to the ammoniation reaction pressure and then returned to the ammoniation reactor. The liquid phase at the bottom of the third vacuum flash tank entered a deamination distillation column from the top, the plate number was 11, and the column top operating pressure was 2MPa. For the sake of energy saving and consumption reduction, no condenser was provided at the top of the deamination distillation column; the top stream of the deamination distillation column was pressurized to the ammoniation reaction pressure and then sent back to the inlet of the ammoniation reactor, and the bottom stream was subjected to subsequent refining.
   The ammoniation reaction product from which ammonia and hydrogen were removed was cut and separated according to different boiling points with hexamethylenediamine as the key component, and orderly subjected to a first separation to obtain a stream containing hexamethyleneimine and a residual stream. Then the residual stream was subjected to a second separation to obtain a hexamethylenediamine product and a stream containing aminohexanol, C12 amine and heavy components, and then the stream containing aminohexanol, C12 amine and heavy components was subjected to a third separation to obtain a stream containing aminohexanol, a stream containing C12 amine, and heavy components. The stream containing aminohexanol and the stream containing hexamethyleneimine were mixed in a weight ratio of 1:1.25 and returned to step (1) as a circulating stream, and the heavy components were withdrawn. Therein, the first separation was carried out in a first distillation column, and the conditions for operating the first distillation column included: a column bottom temperature of 187°C, a column top temperature of 50°C, a reflux ratio of 2.1, a theoretical plate number of 18, and a column top operating pressure of -0.07MPa; the second separation was carried out in a second distillation column, and the conditions for operating the second distillation column included: a column bottom temperature of 195°C, a column top temperature of 131°C, a reflux ratio of 5, a theoretical plate number of 19, and a column top operating pressure of -0.08MPa; the third separation was carried out in a third distillation column, and the conditions for operating the third distillation column included: a column bottom temperature of 338°C, a column top temperature of 87°C, a reflux ratio of 5, a theoretical plate number of 55, and a column top operating pressure of -0.09MPa.
(3) The heavy components obtained from the bottom of the third distillation column in step (2) was sent to an ammonolysis reactor filled with the catalyst for ammonolysis, and ammonia and hydrogen were introduced into the ammonolysis reactor at the same time, wherein the molar ratio of hydrogen:ammonia:heavy components calculated as dihexamethylenetriamine was 5:100:1, the ammonolysis reaction temperature was 170°C, the ammonolysis reaction pressure was 12 MPag, and the liquid space velocity of the stream containing C12 amine was 0.5h⁻¹. The ammonolysis product was returned to step (2).

The weight composition of each stream when the reaction was running stably is shown in Table 1. The molar yield of hexamethylenediamine was 90.69%.

**Table 1**

| | 11 | 12 | 13 | 14 | 15 | 16 | 17 | 19 |
|---|---|---|---|---|---|---|---|---|
| Hydrogen | 0.5 | 0.5 | 0.7 | / | / | / | / | 0.4 |
| Ammonia | 68.6 | 65.8 | 97.9 | / | / | / | / | 85.2 |
| Hexamethylenediamine | / | 8.6 | / | 0.1 | 99.7 | / | / | 4.8 |
| Hexamethyleneimine + Aminohexanol | 16.5 | 16.7 | 0.5' | 81.8 | 0.3 | / | 0.7 | 3.7 |
| Hexanediol | 13.6 | 3.3 | / | 17.0 | / | / | 11.6 | 1.6 |
| C12 amine | 0.1 | 1.0 | / | 0.7 | / | 16.4 | 76.5 | 2.9 |
| Heavy components | / | / | / | / | / | 82.5 | / | 1.0 |
| Others | 0.7 | 4.1 | 0.9 | 0.4 | / | 1.1 | 11.2 | 0.4 |

| | | | | | | | | |
|---|---|---|---|---|---|---|---|---|
| Note: 11: the composition of the stream at the inlet of the ammoniation reactor; 12: ammoniation reaction product; 13: circulating hydrogen and ammonia; 14: circulating stream; 15: hexamethylenediamine product; 16: heavy components; 17: stream containing C12 amine; 19: light components; "/" in the table means that the content was less than 500ppm, or the content was 0. | | | | | | | | |

### Comparative example 1

The method of Example 1 was followed, except that the heavy components obtained were not subjected to ammonolysis. When the reaction was running stably, the molar yield of hexamethylenediamine was 86.5%.

### Comparative example 2

The method of Example 2 was followed, except that the heavy components obtained were not subjected to ammonolysis. When the reaction was running stably, the molar yield of hexamethylenediamine was 87.7%.

### Comparative example 3

The method of Example 3 was followed, except that the heavy components obtained were not subjected to ammonolysis. When the reaction was running stably, the molar yield of hexamethylenediamine was 83.91%.

The preferred embodiments of the invention are described in detail above. However, the invention is not limited thereto. Within the technical concept of the invention, a variety of simple modifications may be made to the technical solution of the invention, including the combination of various technical features in any other suitable manner. These simple modifications and combinations should also be regarded as the contents disclosed by the invention and are within the protection scope of the invention.

## Claims

1. A method for preparing hexamethylenediamine, which comprises the following steps:
(1) under an ammoniation reaction condition, optionally in the presence of a first solvent, subjecting hexanediol and ammonia to an ammoniation reaction to obtain an ammoniation reaction product;
(2) separating the ammoniation reaction product from step (1) to obtain a hexamethylenediamine product and heavy components; and
(3) under an ammonolysis reaction condition, subjecting the heavy components to ammonolysis to obtain an ammonolysis reaction product, and returning the ammonolysis reaction product to step (2).

2. The method according to claim 1, wherein during the ammoniation reaction, the molar ratio of ammonia to hexanediol is 10-90:1, preferably 15-70:1; the ammoniation reaction is carried out in the presence of hydrogen, and the molar ratio of hydrogen to hexanediol is 0.05-25:1, preferably 0.1-10:1.

3. The method according to claim 1 or 2, wherein the temperature of the ammoniation reaction is 100-300°C, preferably 120-250°C; the pressure of the ammoniation reaction is 4-25 MPaG, preferably 6-20 MPaG, and the liquid volume space velocity of the fresh hexanediol is 0.01-15h⁻¹, preferably 0.03-8h⁻¹.

4. The method according to any one of claims 1-3, wherein step (2) further comprises:
separating the ammoniation reaction product to obtain a stream containing hexamethyleneimine and water, and optionally, separating the stream containing hexamethyleneimine and water to obtain a stream containing hexamethyleneimine, and returning at least part of the stream containing hexamethyleneimine to step (1); preferably, the weight ratio of the stream containing hexamethyleneimine returned to step (1) to the fresh hexanediol is 0.1-26:1, more preferably 0.3-24:1; and/or
separating the ammoniation reaction product to obtain a stream containing aminohexanol, and optionally, returning at least part of the stream containing aminohexanol to step (1); preferably, the weight ratio of the stream containing aminohexanol returned to step (1) to the fresh hexanediol is 0.1-26:1, more preferably 0.3-24:1.

5. The method according to any one of claims 1-3, wherein step (2) further comprises:
separating the ammoniation reaction product to obtain a stream containing hexamethyleneimine and water, separating the stream containing hexamethyleneimine and water to obtain a stream containing hexamethyleneimine, and returning at least part of the stream containing hexamethyleneimine to step (1); and
separating the ammoniation reaction product to obtain a stream containing aminohexanol, and returning at least part of the stream containing aminohexanol to step (1);
preferably, the weight ratio of the stream containing hexamethyleneimine to the stream containing aminohexanol returned to step (1) is 0.1-5:1;
preferably, the ratio of the total weight of the stream containing hexamethyleneimine and the stream containing aminohexanol returned to step (1) to the weight of the fresh hexanediol is 0.1-30:1, preferably 0.3-25:1.

6. The method according to claim 4 or 5, wherein the approach for separating the stream containing hexamethyleneimine and water includes at least one of atmospheric distillation, vacuum distillation, pressure swing distillation, azeotropic distillation, membrane separation and centrifugal separation;
preferably, the azeotropic agent used in the azeotropic distillation is at least one of cyclohexane, n-hexane, trimethylpentane, 1-methyl-4-isopropylbenzene, 1,4-dioxane, phenol, cresol, dibutyl ether, diamyl ether and diisoamyl ether.

7. The method according to any one of claims 1-6, wherein the approach for separating the ammoniation reaction product is: subjecting the ammoniation reaction product to a first separation to obtain a stream containing hexamethyleneimine and water, followed by a second separation to obtain the hexamethylenediamine product, and a stream containing aminohexanol and heavy components, and followed by a third separation to obtain a stream containing aminohexanol, and the heavy components;
preferably, the first separation, the second separation and the third separation each independently include at least one of distillation, membrane separation and pressure swing adsorption.

8. The method according to any one of claims 1-7, wherein before the ammoniation reaction product is separated, it is first pre-separated to obtain circulating hydrogen and ammonia, wherein the circulating hydrogen and ammonia are returned to the ammoniation reaction;
preferably, the pre-separation includes at least one of flash evaporation, distillation and stripping.

9. The method according to any one of claims 1-8, wherein step (3) further comprises: dissolving the heavy components in a second solvent before the ammonolysis reaction, and then carrying out the ammonolysis; the second solvent is at least one of tetrahydrofuran, 1,4-dioxane, n-hexane, cyclohexane and tert-butanol; and the weight ratio of the second solvent to the heavy components is 1-20:1.

10. The method according to any one of claims 1-9, wherein in step (3), the ammonolysis is carried out in the presence of hydrogen and ammonia, and the molar ratio of hydrogen:ammonia:heavy components is 0.1-20:10-150:1, wherein the heavy components are calculated as dihexamethylenetriamine.

11. The method according to any one of claims 1-10, wherein the conditions of the ammonolysis reaction include: a reaction temperature of 120-300°C, preferably 150-270°C; a reaction pressure of 9-25MPaG, preferably 10-22MPaG; and a liquid volume space velocity of the heavy components of 0.01-8h⁻¹, preferably 0.05-5h⁻¹.

12. The method according to any one of claims 1-11, wherein
step (1) comprises: under the ammoniation reactions condition, subjecting hexanediol and ammonia to the ammoniation reaction in the first solvent to obtain the ammoniation reaction product;
step (2) comprises: separating the ammoniation reaction product from step (1) to obtain a stream containing hexamethyleneimine, the first solvent and water, the hexamethylenediamine product, a stream containing aminohexanol, and the heavy components, returning at least part of the stream containing aminohexanol to step (1), and then separating the stream containing hexamethyleneimine, the first solvent and water to obtain a stream containing hexamethyleneimine and a first solvent stream, and returning at least part of the stream containing hexamethyleneimine and at least part of the first solvent stream to step (1).

13. The method according to claim 12, wherein during the ammoniation reaction, the molar ratio of ammonia to hexanediol is 10-80:1, preferably 20-45:1; the ammoniation reaction is carried out in the presence of hydrogen, and the molar ratio of hydrogen to hexanediol is 0.05-20:1, preferably 0.2-5:1.

14. The method according to claim 12 or 13, wherein the temperature of the ammoniation reaction is 100-250°C, preferably 130-210°C; the pressure of the ammoniation reaction is 6-18 MPaG, preferably 8-16 MPaG, the liquid volume space velocity of the fresh hexanediol is 0.01-10h⁻¹, preferably 0.05-4h⁻¹.

15. The method according to any one of claims 12-14, wherein the first solvent is at least one of tetrahydrofuran, 1,4-dioxane, n-hexane, cyclohexane and tert-butanol; and/or
the molar ratio of the first solvent to hexanediol is 0.1-10:1.

16. The method according to any one of claims 12-15, wherein step (2) comprises: distilling the stream containing hexamethyleneimine, the first solvent and water in a distillation column to obtain a stream containing hexamethyleneimine at the bottom of the column, and subjecting the stream at the top of the column to membrane separation or pressure swing distillation to obtain a solvent stream and waste water.

17. The method according to any one of claims 1-11, wherein step (1) comprises: under the ammoniation reaction condition, and in the absence of the first solvent, subjecting hexanediol and ammonia to the ammoniation reaction to obtain the ammoniation reaction product;
preferably, the weight ratio of the stream containing hexamethyleneimine returned to step (1) to the fresh hexanediol is 0.1-13:1, preferably 0.3-10:1.

18. The method according to claim 17, wherein in step (1), the molar ratio of ammonia to hexanediol is 18-50:1, preferably 22-38:1, and the molar ratio of hydrogen to hexanediol is 0.08-6:1, preferably 0.4-3:1.

19. The method according to claim 17 or 18, wherein the temperature of the ammoniation reaction is 120-210°C, preferably 130-200°C; the pressure of the ammoniation reaction is 7-17 MPaG, preferably 8-15 MPaG, the liquid volume space velocity of the fresh hexanediol is 0.05-7h⁻¹, preferably 0.09-3.9h⁻¹.
